# EUROPÄISCHE PATENTSCHRIFT

(11) **EP 2 090 225 B1**
(45) Veröffentlichungstag und Bekanntmachung des Hinweises auf die Patenterteilung: **17.08.2011**
(21) Anmeldenummer: 09001807.8
(22) Anmeldetag: 10.02.2009
(51) Int. Cl.: A61B 5/0478, A61B 5/0492, A61B 5/0496

(54) **Anordnung zum Abgreifen von EEG, EOG und EMG Potentialen**
Assembly for measuring EEG, EOG and EMG
Ensemble destiné à la détection de potentiels EEG, EOG et EMG

(30) Priorität: 15.02.2008 DE 202008002129 U
(43) Veröffentlichungstag der Anmeldung: 19.08.2009
(73) Patentinhaber: AGS - Medicare GmbH, 98617 Meiningen (DE)
(72) Erfinder: Wagner, Maximilian E., 77815 Bühl (DE); Wagner, Natascha R., 77815 Bühl (DE)
(74) Vertreter: Hauck Patent- und Rechtsanwälte

(56) Entgegenhaltungen:
- EP-A- 1 493 383
- EP-A- 1 800 600
- WO-A-00/20047
- WO-A-02/100267
- WO-A-2006/122349
- WO-A-2007/041946
- US-A- 6 032 065
- US-A1- 2006 258 930

## Beschreibung

Die vorliegende Erfindung betrifft eine am menschlichen Kopf anbringbare Anordnung zum Abgreifen von durch Aktivitäten des Menschen verursachten elektrischen Potentialen durch Elektroden zu Diagnosezwecken, insbesondere zur Erstellung von Schlaf- und Tagesprofilen, mit den Merkmalen des Oberbegriffs von Patentanspruch 1.

Die Elektroenzephalographie (EEG) ist eine Methode der medizinischen Diagnostik zur Messung der summierten elektrischen Aktivität des Gehirns durch Aufzeichnung der Spannungsschwankungen an der Kopfoberfläche. Ein Elektroenzephalogramm (EEG) ist die grafische Darstellung dieser Schwankungen.

Derartige elektroenzephalographische Messungen werden beispielsweise auch zur automatischen Generierung von Tages- und Schlafprofilen für die Diagnose von Schlaf- und Vigilanzstörungen durchgeführt, wobei die Messungen mit Hilfe eines entsprechende Elektroden tragenden Bandes am menschlichen Kopf vorgenommen werden. So ist beispielsweise aus der DE 100 42 813 A1 eine Anordnung der eingangs beschriebenen Art bekannt, die ein Stirnband aufweist, in dem ein Vorverstärker und drei Elektroden untergebracht sind. Die mittlere Elektrode dient als Referenz, während die beiden äußeren Elektroden eine bipolare Ableitung des frontalen EEG ermöglichen. Das Stirnband ist dabei etwa rechteckförmig ausgebildet.

Zur Ermittlung von derartigen Tages- und Schlafprofilen ist es ferner bekannt, zusätzlich zum EEG noch ein Elektromyogramm (EMG) als Indikator des Muskeltonus und ein Elektrookulogramm (EOG) als Indikator für die Bewegung der Augen zu verwenden und auszuwerten. Zur Durchführung dieser zusätzlichen Messungen finden gesonderte Elektroden Verwendung.

Eine Anordnung mit den Merkmalen des Oberbegriffes von Patentanspruch 1 ist aus der US-A-6 032 065 bekannt. Bei dieser bekannten Anordnung sind die Elektroden auf den beiden Längsschenkeln des Bandes in gleichen Positionen angeordnet, so dass nach dem Anlegen des Bandes gleiche Bereiche auf beiden Gesichtshälften erfasst werden.

Aus der US 2006/258930 A1 ist bekannt, bei derartigen Anordnungen ansteckbare Elektroden zu verwenden.

Der vorliegenden Erfindung liegt die Aufgabe zugrunde, eine Anordnung der eingangs beschriebenen Art zu schaffen, die bei einem einfachen Aufbau einen gegenüber dem Stand der Technik erweiterten Messbereich bietet.

Diese Aufgabe wird erfindungsgemäß bei einer Anordnung der angegebenen Art durch das kennzeichnende Merkmal von Patentanspruch 1 gelöst.

Die erfindungsgemäße Lösung sieht ein einziges Band vor, das sowohl eine EEG-Messung als auch eine EOG- und EMG-Messung ermöglicht. Bei der EOG bzw. Elektrookulographie handelt es sich um ein Messverfahren, bei dem die Bewegung der Augen gemessen wird. Dabei wird die elektrische Spannung gemessen, die zwischen zwei Elektroden auftritt, welche links und rechts (oder oberhalb und unterhalb) des Auges auf der Haut angebracht sind. Diese Anordnung wird durch die erfindungsgemäß ausgebildete Anordnung ermöglicht. Durch Augenbewegungen nähert sich die Vorderseite des Auges der einen Elektrode an, während die Rückseite sich der anderen Elektrode annährt. Dadurch kommt es zu einer Spannungsdifferenz zwischen den Elektroden, die gemessen wird. Hiermit können nicht nur Störungen des Augenbewegungssystems, sondern auch des Gleichgewichtsystems erfasst werden. Bei der Erzeugung von Schlafprofilen dient die EOG-Messung zur Erfassung der REM-Phasen.

Ferner ermöglicht die erfindungsgemäß ausgebildete Anordnung die Durchführung einer Elektromyographie (EMG) bzw. einer elektromyographischen Messung. Hierbei wird die elektrische Muskel-Aktivität gemessen.

Da das erfindungsgemäß ausgebildete Band etwa die Form eines umgedrehten U besitzt (in der Anlegeposition, wenn es in einer Ebene liegt) und die Elektroden in der vorstehend geschilderten Weise befestigt bzw. befestigbar sind, lassen sich bei Anordnung des Bandes auf der Gesichtsseite des menschlichen Kopfes die beiden Elektroden des Querschenkels im Stirnbereich und die Elektroden der Längsschenkel im Augenbereich und im Kinnbereich anbringen. Nach dem Anlegen des Bandes erstreckt sich der Querschenkel quer über die Stirn, während die beiden Längsschenkel seitlich an den Augen vorbei bis zum Kinn verlaufen und im Kinnbereich enden.

Bei der erfindungsgemäß ausgebildeten Anordnung können die Elektroden fest mit dem Band verbunden oder hieran lösbar befestigbar sein. Im zuletzt genannten Fall ist das Band mit Befestigungsmitteln zum lösbaren Anbringen der Elektroden versehen, wobei die Elektroden insbesondere an die Befestigungsmittel angesteckt werden können, beispielsweise mit der Hilfe von Druckknöpfen, wobei das Band und die Elektroden jeweils eine Hälfte eines Druckknopfes tragen. Die Druckknöpfe sind vorzugsweise metallisch ausgebildet, um die gemessenen Potentiale über das entsprechende Leiterbahnen aufweisende Band an zugehörige Auswerteeinrichtungen leiten zu können.

Um entsprechende Messungen seitlich oberhalb und unterhalb der Augen sowie in verschiedenen Kinnbereichen durchführen zu können, sind bei der erfindungsgemäß ausgebildeten Anordnung die Elektroden auf den beiden Längsschenkeln versetzt zueinander angeordnet. Die EMG-Elektroden sind zweckmäßigerweise an den Enden der Längsschenkel angeordnet, wobei vorzugsweise die Längsschenkel verschieden lang ausgebildet sind. Der kürzere Längsschenkel, bei dem die Elektroden im Vergleich zu den Elektroden des anderen Schenkels näher am Querschenkel, d. h. nach dem Anlegen des Bandes im Gesicht weiter oben, angeordnet sind, ermöglicht dabei Messungen oberhalb des Auges und im oberen Kinnbereich, während der andere Schenkel Messungen unterhalb des Auges und im unteren Kinnbereich ermöglicht.

Die Anordnung ist ferner vorzugsweise im Bereich der Elektroden mit Klebepads versehen/versehbar. Beim Anlegen des Bandes werden das Band bzw. die Elektroden mit Hilfe der Klebepads an den entsprechenden Kopf/Gesichtsbereichen fixiert. Hierdurch wird der erforderliche Hautkontakt der EEG-, EOG- und EMG-Elektroden erzielt.

Für jede Messung weist daher das erfindungsgemäß ausgebildete Band zwei Elektroden auf, so dass die Aufzeichnung von drei einzelnen bipolar geschalteten Kanälen möglich wird. Die entsprechenden Messpositionen sind durch das Band vorgegeben. Durch das Anlegen des Bandes wird die Applizierung der einzelnen Elektroden erheblich vereinfacht, und Fehler werden weitgehend vermieden. Die jeweiligen Bänder können verschiedene Größen aufweisen und somit patientenspezifisch ausgebildet sein. Sie bestehen vorzugsweise aus einem geeigneten Kunststoffmaterial, das desinfizierbar ist und somit mehrfach verwendet werden kann.

Das erfindungsgemäß ausgebildete Band, das vorzugsweise, wie vorstehend erwähnt, aus einem flexiblen Kunststoffmaterial besteht, weist vorzugsweise integrierte Leiterbahnen auf, die zu den jeweiligen Elektroden bzw. Befestigungsmitteln für die Elektroden führen. Die Anordnung weist zweckmäßigerweise etwa mittig am Querschenkel einen Anschluss bzw. eine Schnittstelle zu einer Auswerteeinrichtung auf, zu dem bzw. der die einzelnen Leiterbahnen von den Elektroden führen. Der Bereich des Anschlusses bzw. der Schnittstelle ist vorzugsweise verstärkt, um entsprechende Anschlusselemente anbringen zu können. So kann beispielsweise ein Verstärker/Vorverstärker am Anschlussbereich angebracht bzw. an diesem anbringbar sein. Der Verstärker/Vorverstärker ist dabei vorzugsweise auf den verstärkten Anschluss aufsteckbar.

Die Verbindung zwischen der erfindungsgemäßen Anordnung und der Auswerteeinrichtung kann über geeignete Leitungen aber auch drahtlos mit Hilfe von geeigneten Sende- bzw. Empfangseinrichtungen erfolgen.

Die Erfindung wird nachfolgend anhand eines Ausführungsbeispiels in Verbindung mit der Zeichnung im Einzelnen erläutert. Es zeigen:
- Figur 1: eine Draufsicht auf eine am menschlichen Kopf anbringbare Anordnung zum Abgreifen von elektri- schen Potentialen in einer ebenen Lage in der Anlageposition; und
- Figur 2: die Anordnung der Figur 1 in einem an einem menschlichen Kopf angebrachten Zustand.

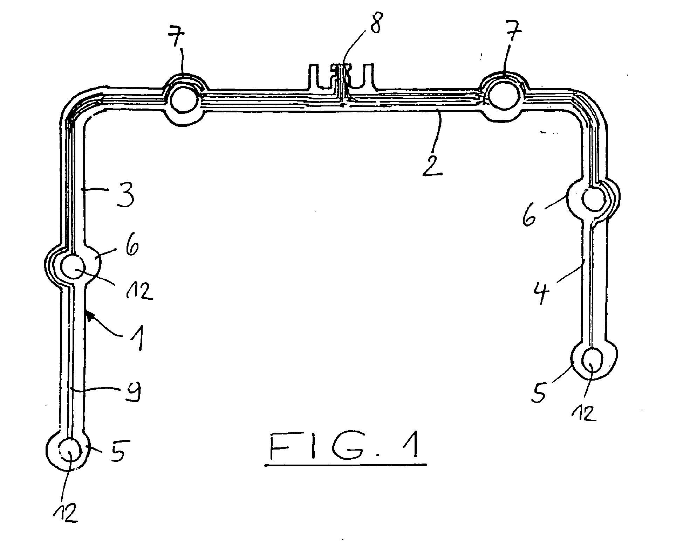

Die in Figur 1 in der Draufsicht und in ebener Lage dargestellte Anordnung umfasst ein Kunststoffband 1, das etwa die Form eines umgedrehten U besitzt und einen Querschenkel 2 sowie zwei sich daran anschließende Längsschenkel 3 und 4 aufweist. Der in Figur 1 links dargestellte Längsschenkel 3 ist dabei länger ausgebildet als der in Figur 1 rechts dargestellte Längsschenkel 4. Am Band 1 sind in bestimmten Positionen Elektroden (nicht gezeigt) anbringbar, um EEG-, EOG- und EMG-Messungen durchzuführen. Die jeweiligen Elektrodenpositionen sind mit 5, 6 und 7 gekennzeichnet. Im Einzelnen befinden sich auf dem Querschenkel zwei Positionen 7 für EEG-Elektroden. Auf den beiden Längsschenkeln 3 und 4 befinden sich jeweils zwei Positionen 6 für EOG-Elektroden und zwei Positionen 5 für EMG-Elektroden. Die Positionen 5 befinden sich jeweils am Ende eines Längsschenkels.

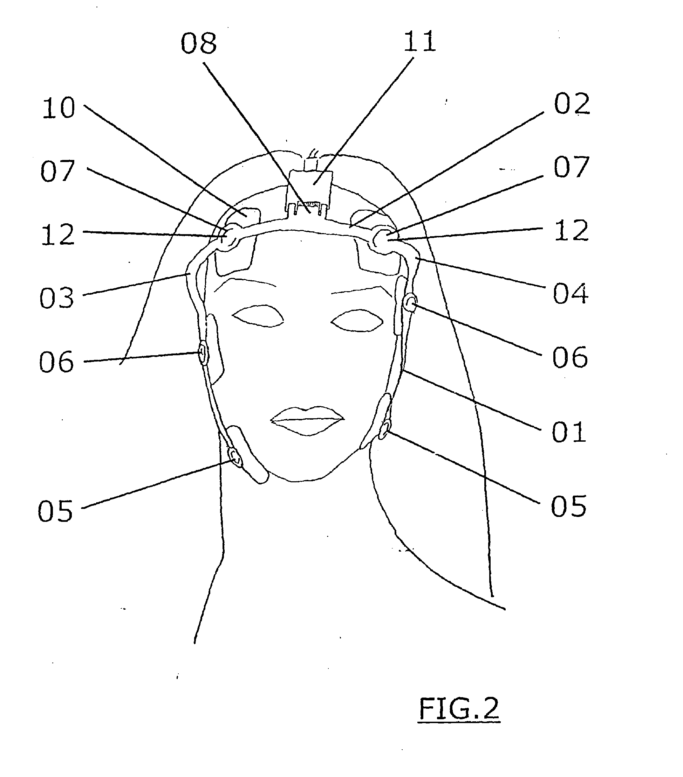

An den Elektrodenpositionen sind jeweils Druckknöpfe 12 angeordnet, von denen die Rückseite in Figur 1 gezeigt ist. In diese Druckknöpfe 12 sind mit Klebepads 10 (in Figur 2 gezeigt) ausgestattete handelsübliche Elektroden einklippbar.

Die Druckknöpfe 12 bestehen aus Metall und sind daher elektrisch leitend. Sie stehen mit in das Kunststoffband 1 integrierten Leiterbahnen 9 in Verbindung, wobei jeweils zwei Leiterbahnen 9 einem Druckknopf 12 und damit einer Elektrode zugeordnet sind. Die entsprechenden Leiterbahnen erstrecken sich bis zu einem etwa mittig im Querschenkel 2 angeordneten Anschluss 8 (Schnittstelle),der gegenüber dem übrigen Band verstärkt ausgebildet ist, um einen geeigneten Anschluss an eine Auswerteeinrichtung zu ermöglichen. Beispielsweise kann am Anschluss 8 ein Verstärker/Vorverstärker fixiert werden.

Zu jeder Elektrodenposition führen somit zwei Leiterbahnen 9, wobei die Leiterbahnen auf ihrem Wege zum Anschluss 8 entsprechende weitere Elektrodenpositionen umgehen. So sind beispielsweise die Leiterbahnen 9 von der Elektrodenposition 5 etwa halbkreisförmig um die Elektrodenpositionen 6 und 7 herumgeführt.

Figur 2 zeigt die Anordnung bzw. das Band 1 (Gesichtsmaske) im angelegten Zustand. Die Befestigung des Bandes erfolgt mit Hilfe der Klebepads 10, die mit den entsprechenden Elektroden (nicht gezeigt) versehen sind. Im am Kopf befestigten Zustand verläuft der Querschenkel 2 des Bandes quer über die Stirn, während sich die beiden Längsschenkel 3 und 4 seitlich am Kopf nach unten erstrecken. Dabei ist in Figur 2 der kürzere Längsschenkel 4 rechts dargestellt. Man erkennt, dass die beiden Elektrodenpositionen 5, 6 an diesem Längsschenkel gegenüber den Elektrodenpositionen des anderen Längsschenkels 3 versetzt sind, d. h. in Figur weiter oben liegen. Die EOG-Elektrode des in Figur 2 kürzeren Längsschenkels 4 befindet sich daher etwas oberhalb des zugehörigen Auges, während die EOG-Elektrode des in der Figur längeren Schenkels unterhalb des zugehörigen Auges angeordnet ist. Auch die EMG-Elektroden sind auf verschiedenen Höhen des Kinnbereiches angeordnet.

Figur 2 zeigt ferner, dass ein geeigneter Verstärker 11 auf den Anschluss 8 aufgesteckt ist. Vom Verstärker führt eine entsprechende Leitung zur Auswerteeinrichtung.

Mit der hier dargestellten Anordnung lassen sich gleichzeitig elektroenzephalographische, elektrookulographische und elektromyographische Messungen zum automatischen Generieren von Tages- und Schlafprofilen für Diagnosezwecke durchführen.

## Patentansprüche

1. Am menschlichen Kopf anbringbare Anordnung zum Abgreifen von durch Aktivitäten des Menschen verursachten elektrischen Potentialen durch Elektroden zu Diagnosezwecken, insbesondere zur Erstellung von Schlaf- und Tagesprofilen, die als die Elektroden tragendes flexibles Band (1) ausgebildet und mit zwei eine bipolare Ableitung des frontalen EEG ermöglichenden Elektroden versehen ist, wobei das Band (1) etwa die Form eines umgedrehten U mit einem Querschenkel (2) und zwei Längsschenkeln (3, 4) besitzt, die EEG-Elektroden mit Abstand voneinander am Querschenkel (2) vorgesehen sind und die Längsschenkel (3, 4) mit je einer EOG-Elektrode und einer EMG-Elektrode im Abstand voneinander versehen sind, **dadurch gekennzeichnet, dass** die zugehörigen Elektroden auf den beiden Längsschenkeln (3, 4) versetzt zueinander angeordnet sind.

2. Anordnung nach Anspruch 1, **dadurch gekennzeichnet, dass** das Band (1) Befestigungsmittel zur lösbaren Anbringung der Elektroden aufweist.

3. Anordnung nach einem der vorangehenden Ansprüche, **dadurch gekennzeichnet, dass** die EMG-Elektroden an den Enden der Längsschenkel (3, 4) angeordnet sind.

4. Anordnung nach einem der vorangehenden Ansprüche, **dadurch gekennzeichnet, dass** die Längsschenkel (3, 4) verschieden lang ausgebildet sind.

5. Anordnung nach Anspruch 2, **dadurch gekennzeichnet, dass** die Elektroden an die Befestigungsmittel ansteckbar sind.

6. Anordnung nach Anspruch 5, **dadurch gekennzeichnet, dass** die Elektroden mit Hilfe von Druckknöpfen (12) an das Band (1) ansteckbar sind.

7. Anordnung nach einem der vorangehenden Ansprüche, **dadurch gekennzeichnet, dass** sie im Bereich der Elektroden mit Klebepads (10) versehen ist.

8. Anordnung nach einem der vorangehenden Ansprüche, **dadurch gekennzeichnet, dass** das Band (1) integrierte Leiterbahnen (9) aufweist, die zu den jeweiligen Elektroden bzw. Befestigungsmitteln führen.

9. Anordnung nach einem der vorangehenden Ansprüche, **dadurch gekennzeichnet, dass** sie etwa mittig am Querschenkel (2) einen Anschluss (8) zu einer Auswerteeinrichtung aufweist.

10. Anordnung nach Anspruch 9, **dadurch gekennzeichnet, dass** der Bereich des Anschlusses (8) verstärkt ist.

11. Anordnung nach Anspruch 9 oder 10, **dadurch gekennzeichnet, dass** ein Verstärker (11) am Anschlussbereich angebracht ist.

## Claims

1. An assembly for measuring electrical potentials caused by activities of a human being by electrodes for diagnostic purposes, especially for establishing sleep profiles and day profiles, said assembly being adapted to be placed at the human head and being formed as flexible band (1) bearing the electrodes and being provided with two electrodes enabling a bipolar derivation of the frontal EEG, wherein the band (1) has approximately the shape of a reversed U with a cross leg (2) and two longitudinal legs (3, 4), the EEG electrodes being provided at the cross leg (2) in a spaced condition, and the longitudinal legs (3, 4) being provided with a respective EOG electrode and a respective EMG electrode in a spaced condition, **characterized in that** the associated electrodes are disposed on the two longitudinal legs (3, 4) offset with respect to one another.

2. The assembly according to claim 1, **characterized in that** the band (1) includes fixation means for the removable placement of the electrodes.

3. The assembly according to one of the preceding claims, **characterized in that** the EMG electrodes are disposed at the ends of the longitudinal legs (3, 4).

4. The assembly according to one of the preceding claims, **characterized in that** the longitudinal legs (3, 4) have a different length.

5. The assembly according to claim 2, **characterized in that** the electrodes are adapted to be clipped to the fixation means.

6. The assembly according to claim 5, **characterized in that** the electrodes are adapted to be clipped to the band (1) by means of press buttons (12).

7. The assembly according to one of the preceding claims, **characterized in that** it is provided with adhesive pads (10) in the region of the electrodes.

8. The assembly according to one of the preceding claims, **characterized in that** the band (1) has integrated conductor tracks (9) leading to the respective electrodes or fixation means.

9. The assembly according to one of the preceding claims, **characterized in that** it has a connection (8) to an evaluation device approximately centrally at the cross leg (2).

10. The assembly according to claim 9, **characterized in that** it is reinforced in the region of the connection (8).

11. The assembly according to claim 9 or 10, **characterized in that** an amplifier (11) is mounted at the connection region.

## Revendications

1. Ensemble pouvant être disposé sur la tête d'un être humain, destiné à détecter des potentiels provoqués par les activités de l'être humain grâce à des électrodes afin d'établir un diagnostic, en particulier des profils nocturnes et diurnes, conçu comme une bande flexible (1) portant les électrodes et pourvu de deux électrodes permettant une dérivation bipolaire de l'EEG frontal, la bande (1) présentant approximativement la forme d'un U inversé comprenant une branche transversale (2) et deux branches longitudinales (3, 4), les électrodes EEG étant disposées à distance les unes des autres sur la branche transversale (2) et les branches longitudinales (3, 4) étant respectivement pourvues d'une électrode EOG et d'une électrode EMG distantes l'une de l'autre, **caractérisé en ce que** les électrodes associées sont disposées sur les deux branches longitudinales (3, 4) en étant décalées l'une de l'autre.

2. Ensemble selon la revendication 1, **caractérisé en ce que** la bande (1) comprend des moyens de fixation destinés à l'agencement amovible des électrodes.

3. Ensemble selon l'une quelconque des revendications précédentes, **caractérisé en ce que** les électrodes EMG sont disposées aux extrémités des branches longitudinales (3, 4).

4. Ensemble selon l'une quelconque des revendications précédentes, **caractérisé en ce que** les branches longitudinales (3, 4) sont de différentes longueurs.

5. Ensemble selon la revendication 2, **caractérisé en ce que** les électrodes peuvent être branchées aux moyens de fixation.

6. Ensemble selon la revendication 5, **caractérisé en ce que** les électrodes peuvent être branchées à la bande (1) à l'aide de boutons-poussoirs (12).

7. Ensemble selon l'une quelconque des revendications précédentes, **caractérisé en ce qu'**il est pourvu de tampons adhésifs (10) dans la zone des électrodes.

8. Ensemble selon l'une quelconque des revendications précédentes, **caractérisé en ce que** la bande (1) comprend des tracés conducteurs (9) intégrés menant aux électrodes respectives ou aux moyens de fixation.

9. Ensemble selon l'une quelconque des revendications précédentes, **caractérisé en ce qu'**il comprend un raccordement (2) à un dispositif d'analyse approximativement au centre sur la branche transversale.

10. Ensemble selon la revendication 9, **caractérisé en ce que** la zone du raccordement (8) est renforcée.

11. Ensemble selon la revendication 9 ou 10, **caractérisé en ce qu'**un amplificateur (11) est disposé sur la zone de raccordement.
